# EUROPEAN PATENT APPLICATION

(11) **EP 4 692 108 A1**
(43) Date of publication of application: **11.02.2026**
(21) Application number: 24785165.2
(22) Date of filing: 02.04.2024
(51) Int. Cl.: C07K 7/06, A61K 8/64, A61Q 5/00

(54) **PEPTIDE WITH ACTIVITY OF PROMOTING HAIR GROWTH OR MITIGATING HAIR LOSS AND USE THEREOF**

(30) Priority: 05.04.2023 KR 20230044927
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR)
(74) Representative: Potter Clarkson
(86) International application number: PCT/KR2024/004243
(87) International publication number: WO 2024/210460

(57) **Abstract**

The present application relates to a peptide having hair growth-promoting activity, and use thereof, and provides a peptide consisting of the amino acid sequence of SEQ ID NO: 1, a cosmetic composition for promoting hair growth and improving hair loss including the peptide, and a pharmaceutical composition for preventing or treating hair loss including the peptide.

## Description

### Technical Field

The present disclosure relates to a peptide having hair growth-promoting or hair loss-inhibiting activity and its use.

### Background Art

As an aging society develops, the number of people concerned about hair loss is increasing, and although this was previously a major problem for middle-aged men, recently interest in hair loss prevention, hair growth, etc., is increasing among younger generations and women as well. Meanwhile, hair loss has been recognized as a series of aging phenomena, but recently it has been revealed that hair loss progresses due to various causes such as genetic factors, stress, westernized eating habits, nutritional imbalance, and changes in social activities.

Currently, drugs approved by the U.S. FDA for hair growth effects are minoxidil (6-Amino-1,2-dihydro-1-hydroxy-2-imino-4-phenoxypyrimidine) (U.S. Patent No. 3,382,247) and finasteride (U.S. Patent No. 5,215,894). In the case of minoxidil, it was developed in the early 1970s as a vasodilator for the treatment of hypertension, but as hypertrichosis was reported as a side effect, it has been used as a hair growth promoter, exhibiting the effects of thickening hair by enlarging pores and increasing the diameter of hair shafts. In addition, in the case of finasteride, it was developed as a therapeutic agent for prostatic hypertrophy and is currently used as a therapeutic agent for hair loss, slowing the progression of hair loss and exhibiting hair growth effects.

However, in the case of minoxidil, side effects such as weight gain, edema, dermatitis, and increased heart rate have been reported, and in the case of finasteride, continuous administration is required, and side effects such as sexual dysfunction in men and birth defects in pregnant women occur, and therefore the development of a hair loss therapeutic agent without side effects is still in demand.

Against this technical background, multifaceted studies for improving hair loss through mechanisms such as hormone regulation and metabolic regulation are being conducted (Korean Laid-Open Patent Publication No. 2002-0005332), but progress is still insufficient.

### Disclosure of Invention

### Technical Problem

One aspect is to provide a peptide consisting of the amino acid sequence of SEQ ID NO: 1.

Another aspect is to provide a cosmetic composition for improving hair loss or promoting hair growth, including the peptide.

Another aspect is to provide a pharmaceutical composition for preventing or treating hair loss, including the peptide.

Other objectives and advantages of the present application will become apparent from the following detailed description, together with the appended claims and drawings. Any matter not described herein will be sufficiently recognized and inferred by those skilled in the art of the present application or a similar art, and the description is hereby omitted.

### Solution to Problem

Each of the descriptions and embodiments disclosed in the present application may be applied to other descriptions and embodiments. Accordingly, all combinations of the various elements disclosed in the present application fall within the scope of the present application. Furthermore, the scope of the present application is not to be considered limited by the specific description set forth below.

One aspect provides a peptide consisting of the amino acid sequence of SEQ ID NO: 1.

As used herein, the term "peptide" may refer to a linear molecule formed by combining amino acid residues to each other by peptide bonds. The above peptide may be prepared according to chemical synthesis methods known in the art, particularly solidphase synthesis techniques (Merrifield, J. Amer. Chem. Soc. 85:2149-54 (1963)) or liquidphase synthesis techniques (US Patent No. 5,516,891).

As a result of diligent efforts to develop a peptide with biologically effective activity, the inventors of the present disclosure have identified a peptide consisting of the amino acid sequence of SEQ ID NO: 1. More specifically, the biologically effective activity may be exhibit any one or more selected from the group consisting of: (a) promotion of proliferation of human hair follicle dermal papilla cells; (b) promotion of proliferation of human hair follicle outer root sheath cells; (c) promotion of proliferation of human hair follicle germinal matrix cells; (d) activation of β-catenin, for example, increased expression of β-catenin and/or translocation of β-catenin from the cytoplasm to the nucleus; (e) increased expression of Lef-1 (lymphoid enhancer-binding factor-1), Cyclin D1, or c-Myc; (f) promotion of differentiation of human hair follicle stem cells; and (g) inhibition of expression of DKK-1 (Dickkopf-related protein 1).

Therefore, the above peptide may be used for purposes such as promoting hair growth or improving hair loss, preventing or treating hair loss, and improving damaged hair.

The peptide may have a protecting group bound to the N- or C-terminus of the peptide to acquire chemical stability, enhanced pharmacological properties (half-life, absorption, titer, effect, etc.), altered specificity (for example, broad spectrum of biological activity), or reduced antigenicity. Accordingly, the peptide may be used to include both the peptide itself and a protected derivative thereof in which a protecting group is introduced. In an embodiment, an N-terminus of the peptide may be bound with any one protecting group selected from the group consisting of an acetyl group, a fluoreonylmethoxycarbonyl group, a formyl group, a palmitoyl group, myristyl group, stearyl group, butoxycarbonyl group, allyloxycarbonyl group, and polyethylene glycol (PEG); and/or the C-terminus of the peptide may be bound with any one protecting group selected from the group consisting of an amino group(-NH₂), a tertiary alkyl group, and an azide (-NHNH₂). Additionally, the peptide may optionally further include a targeting sequence, a tag, labeled residues, or an amino acid sequence prepared for the specific purpose of increasing the half-life or peptide stability.

The above peptide is artificially synthesized, or non-naturally occurring or engineered, and the "non-naturally occurring or engineered" refers to a state that is not in its natural state, but is created by artificial modification. Here, the artificial modification may include artificially synthesizing an amino acid sequence by mimicking a plurality of amino acid structures, or manipulating it to acquire chemical stability, enhanced pharmacological properties, altered specificity, or reduced antigenicity as described above.

As used herein, the term "stability" may refer to not only in vivo stability, which protects the peptide from attack by proteolytic enzymes in vivo, but also storage stability (for example, room temperature storage stability).

Another aspect provides a cosmetic composition for promoting hair growth or improving hair loss, including a peptide consisting of the amino acid sequence of SEQ ID NO: 1.

In the above description of the peptide, the terms or elements referred to are the same as those already mentioned.

As used herein, the term "improve" may refer to any act that at least reduces a parameter associated with the alleviation or treatment of a condition, for example the severity of a symptom.

As used herein, the term "hair loss improvement" may comprehensively refer to a process or effect of treating, alleviating, or mitigating a hair loss condition, and may refer to, for example, all actions that inhibit the progression of hair loss, such as promoting the proliferation or activation of hair follicle dermal papilla cells, or inhibiting the expression of hair loss-inducing factors such as DKK-1, etc. The above hair follicle dermal papilla cells are cells constituting the hair follicle that affect the thickness and growth of hair, and activation of these cells affects the hair cycle, and through this mechanism, an effect of improving hair loss may be induced.

As used herein, the term "hair growth promotion" may refer to any action that increases hair growth from a hair follicle, and may refer to any action that increases the total amount of hair, for example, the effect of promoting proliferation of hair follicle cells, activation of hair follicle cells, or growth of hair follicle cells, etc. Specifically, the hair cycle may be divided into the growth phase (anagen) in which hair grows, the regression phase (catagen) in which growth ends and the hair bulb shrinks, the resting phase (telogen) in which the dermal papilla ceases activity and the hair remains on the scalp, and the active phase in which the dermal papilla begins activity or produces new hair, causing old hair to fall out. In the above hair cycle, dermal papilla cells are connected to capillaries and sensory nerves, which supply oxygen and nutrients to germinal matrix cells, and the germinal matrix cells surround dermal papilla cells, playing a role in determining the rate of the anagen phase through continuous cell division and proliferation. Additionally, the outer root sheath cells are located in the area that is in contact with the basal layer of the epidermis, and play a role in protecting the hair until keratinization is complete.

Although prior functional peptides have effective biological activity, they have shown disadvantages such as not being able to effectively enter target tissue or cell due to the size of the peptide itself, or disappearing from the body in a short period of time due to their short half-life. In contrast, the cosmetic composition according to an example includes, as an active ingredient, a peptide consisting of 10 or fewer amino acids, and accordingly, the penetration rate, etc., of the active ingredient into the skin is very excellent, and hair growth may be promoted and hair loss may be effectively inhibited, for example, when applied topically to the skin.

According to an example, it has been shown to promote proliferation or activation of hair follicle dermal papilla cells; promoting proliferation or activation of hair follicle outer root sheath cells; promoting proliferation or activation of hair follice germinal matrix cells; activating β-catenin; increasing expression of Lef-1, Cyclin D1, or c-Myc; promoting differentiation of hair follicle stem cells; and inhibiting expression of hair loss-inducing factors such as DKK-1, etc. Thus, the above peptide may be utilized as an active ingredient of a cosmetic composition for promoting hair growth and improving hair loss.

The cosmetic composition may include, a cosmetically effective amount of the peptide; and/or a cosmetically acceptable carrier, but is not limited thereto.

As used herein, the term "cosmetically effective amount" may refer to an amount sufficient to achieve the hair loss improvement or hair growth promotion effect of the cosmetic composition.

The weight ratio between the peptide and a cosmetically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

The cosmetic composition may be prepared in any formulation commonly prepared in the art, for example, may be formulated as a solution, a suspension, an emulsion, a paste, a gel, a cream, a lotion, a powder, a soap, a surfactant-containing cleanser, an oil, a powder foundation, an emulsion foundation, a wax foundation, and a spray, etc., but is not limited thereto. For example, the cosmetic composition may be prepared in the form of a softening lotion, nutrition lotion, nutrition cream, massage cream, essence, eye cream, cleansing cream, cleansing foam, cleansing water, mask, spray or powder.

If the formulation of the cosmetic composition is a paste, cream or gel, the carrier component may be an animal oil, vegetable oil, wax, paraffin, starch, tragacanth, cellulose derivative, polyethylene glycol, silicone, bentonite, silica, talc or zinc oxide, etc.

If the formulation of the cosmetic composition is a powder or spray, lactose, talc, silica, aluminum hydroxide, calcium silicate or polyamide powder may be used as a carrier component and, for example, if the formulation is a spray, it may additionally include a propellant such as chlorofluorohydrocarbon, propane/butane or dimethyl ether.

If the formulation of the cosmetic composition is a solution or emulsion, a solvent, solubilizer or emulsifier is used as a carrier component and may include, for example, water, ethanol, isopropanol, ethyl carbonate, ethyl acetate, benzyl alcohol, benzyl benzoate, propylene glycol, 1,3-butyl glycol oil, glycerol aliphatic esters, polyethylene glycol or fatty acid esters of sorbitan.

If the formulation of the cosmetic composition is a suspension, the carrier component may include a liquid diluent such as water, ethanol or propylene glycol, a suspending agent such as ethoxylated isostearyl alcohol, polyoxyethylene sorbitol esters and polyoxyethylene sorbitan esters, microcrystalline cellulose, aluminum methahydroxide, bentonite, agar or tragacanth, etc.

If the formulation of the cosmetic composition is a surfactant-containing cleanser, the carrier component may include aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, lanolin derivative, or ethoxylated glycerol fatty acid ester, etc.

The peptide may be incorporated into a nanosome or a nanoparticle to further improve skin penetration or stability issues. For example, the nanosome may be prepared by a microfluidizer using lecithin as a raw material, and may be incorporated in lecithin particles. Any method of preparing the nanosome may be used as long as it is known. The size of the nanosome particle is preferably 30 to 200 nm. If the size of the nanosome particle is less than 30 nm, skin penetration may progress very quickly, resulting in skin side effects, and if the size is greater than 200 nm, skin penetration may not be easy, making it difficult to achieve the effect of using the nanosome structure.

The ingredients included in the cosmetic composition include, in addition to peptides and carrier components as active ingredients, components commonly used in cosmetic compositions, and may include, for example, common adjuvants such as an antioxidant, stabilizer, solubilizer, vitamin, pigment, and fragrance.

The content of the peptide as an active ingredient contained in the cosmetic composition may be selected appropriately without limitation depending on the form of the product, the desired use, etc., and may be added, for example, from 0.01 to 15 wt% of the total weight of the cosmetic composition. Additionally, for example, the cosmetic composition may include the peptide in an amount of from 1.0 wt% to 3.0 wt%, preferably from 2.0 wt% to 3.0 wt%, based on the total weight.

Another aspect provides a method of improving hair loss or promoting hair growth, including applying a cosmetic composition including a peptide consisting of the amino acid sequence of SEQ ID NO: 1 as an active ingredient to the skin of a subject.

In the above description of the cosmetic composition, the terms or elements referred to are the same as those already mentioned.

As used herein, the terms "applying," "administering," and "applying" are used interchangeably and may refer to causing at least partial localization of a composition according to an example to a desired site, or to placing a composition according to an example into a subject by route of administration.

Another aspect provides a pharmaceutical composition for preventing or treating hair loss, including a peptide consisting of the amino acid sequence of SEQ ID NO: 1 as an active ingredient.

In the above description of the peptide, the terms or elements referred to are the same as those already mentioned.

As used herein, the term "prevention" refers to any act of suppressing or delaying the onset of a disease by administering the composition.

As used herein, the term "treatment" refers to any form of treatment that provides an effect to a subject suffering from a disease or at risk of developing a disease, including improving the condition of the subject (for example, one or more symptoms), delaying the progression of the disease, delaying the onset of symptoms, or slowing the progression of symptoms, etc. Therefore, the above "treatment" and "prevention" are not intended to refer to a cure or complete elimination of symptoms.

The above "subject" refers to a target requiring treatment for a disease, and more specifically, a mammal such as a human or non-human primate, mouse, dog, cat, horse, cow, and the like.

"Hair loss," which is a disease targeted for prevention or treatment by the pharmaceutical composition, refers to a state in which hair is absent in an area where hair should normally exist, and specifically, it may refer to the loss of terminal hair (thick, black hair) on the scalp. The causes of hair loss are not limited, and it may be induced by various genetic factors, hormonal imbalances, psychological stress, exposure to air pollution, dietary habits such as consumption of processed foods, and other environmental influences. For example, hair loss may be caused by hereditary androgenetic alopecia (baldness), alopecia areata, tinea capitis caused by fungal infection, telogen effluvium, trichotillomania, and hair growth disorders, etc., and scarring alopecia may include hair loss due to lupus, lichen planopilaris, folliculitis decalvans, burns, or trauma.

According to an example, the peptide showed effects of promoting proliferation or activation of hair follicle dermal papilla cells; promoting proliferation or activation of hair follicle outer root sheath cells; promoting proliferation or activation of hair follice germinal matrix cells; activating β-catenin; increasing expression of Lef-1, Cyclin D1, or c-Myc; promoting differentiation of hair follicle stem cells; and inhibiting expression of hair loss-inducing factors such as DKK-1, etc. Thus, the above peptide may be used as an active ingredient of a pharmaceutical composition for preventing or treating hair loss.

The pharmaceutical composition may include, a pharmaceutically effective amount of the peptide; and/or a pharmaceutically acceptable carrier, but is not limited thereto.

As used herein, the term "pharmaceutically effective amount" refers to an amount sufficient to achieve the hair loss prevention or treatment efficacy of the pharmaceutical composition.

The pharmaceutically acceptable carrier is commonly used in formulations and include, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, etc., but is not limited thereto. Suitable pharmaceutically acceptable carriers and formulations are described in detail in Remington's Pharmaceutical Sciences (19th ed., 1995).

The weight ratio between the peptide and a pharmaceutically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

In addition to the above ingredients, the pharmaceutical composition may additionally include, lubricants, humectant, sweetener, flavoring agent, emulsifier, suspending agent, preservative, etc., but is not limited thereto.

The pharmaceutical composition may be administered orally or parenterally, preferably parenterally, and if parenterally administered, may be administered by, but not limited to, intramuscular injection, intravenous injection, subcutaneous injection, intraperitoneal injection, topical administration, transdermal administration, etc.

The dosage of the pharmaceutical composition may be, but is not limited to, 0.0001 to 1000 µg (micrograms), 0.001 to 1000 µg, 0.01 to 1000 µg, 0.1 to 1000 µg, or 1.0 to 1000 µg per day, and may be varied by factors such as method of formulation, mode of administration, patient age, weight, sex, medical condition, food, time of administration, route of administration, rate of excretion, and response sensitivity.

The pharmaceutical composition may be formulated, with a pharmaceutically acceptable carrier and/or excipient, according to a method that could be readily practiced by a person skilled in the art to which the present disclosure belongs, and the composition may be prepared into a unit dosage form or may be contained in a multi-dose container.

The formulation may be in the form of a solution, suspension or emulsion in an oil or aqueous medium, or in the form of an extract, powder, granule, tablet or capsule, and may additionally include a dispersant and/or stabilizer.

Another aspect provides a method of preventing or treating hair loss, including administering to a subject a pharmaceutical composition including a therapeutically effective amount of a peptide consisting of the amino acid sequence of SEQ ID NO: 1 as an active ingredient.

In the above description of the pharmaceutical composition, the terms or elements referred to are the same as those already mentioned.

Another aspect provides a food composition for preventing hair loss or promoting hair growth, including a peptide consisting of the amino acid sequence of SEQ ID NO: 1 as an active ingredient.

In the above description of the peptide, the terms or elements referred to are the same as those already mentioned.

The content of the peptide as an active ingredient contained in the food composition may be appropriately selected without limitation depending on the form of the food, the desired use, etc., and may be added, for example, from 0.01 to 15 wt% of the total weight of the food. Additionally, for example, the health drink composition may be added at a rate of 0.02 to 10 g, preferably 0.3 to 1 g, based on 100 ml.

### Advantageous Effects of Invention

According to an aspect, the peptide may be applied to hair growth promotion, and the like by (a) promoting proliferation of human hair follicle dermal papilla cells (HHFDPC), (b) promoting proliferation of human hair follicle outer root sheath cells (HHFORSC), (c) promoting proliferation of human hair follicle germinal matrix cells (HHFGMC), (d) activating β-catenin, (e) increasing expression of Lef-1, Cyclin D1, or c-Myc, or (f) promoting differentiation of human hair follicle stem cells (HHFSC), thereby promoting proliferation or activation of hair follicle cells or promoting expression of hair follicle stem cells.

According to another aspect, the peptide may exhibit the ability to inhibit expression of hair loss factors and may be applied to inhibit hair loss, etc.

### Brief Description of Drawings

FIG. 1 shows the results of measuring the proliferation-promoting effect of a peptide consisting of the amino acid sequence of SEQ ID NO: 1 on human hair follicle dermal papilla cells.
FIG. 2 shows the results of measuring the proliferation-promoting effect of a peptide consisting of the amino acid sequence of SEQ ID NO: 1 on human hair follicle outer root sheath cells.
FIG. 3 shows the results of measuring the proliferation-promoting effect of a peptide consisting of the amino acid sequence of SEQ ID NO: 1 on human hair follicle germinal matrix cells.
FIG. 4 shows the results of measuring the effect of a peptide consisting of the amino acid sequence of SEQ ID NO: 1 on promoting the proliferation mechanism of hair follicle cells.
FIG. 5 shows the results confirming that a peptide consisting of the amino acid sequence of SEQ ID NO: 1 increases the expression of Lef-1, Cyclin D1, and c-Myc, which are factors related to the activity and growth of hair follicle cells.
FIG. 6 is a result confirming that the expression of stem cell markers is reduced due to differentiation induction by a peptide consisting of the amino acid sequence of SEQ ID NO: 1.
FIG. 7 is a result confirming that the expression of a differentiation marker increases due to differentiation induction by a peptide consisting of the amino acid sequence of SEQ ID NO: 1.
FIG. 8 shows the results of measuring the inhibitory effect of a peptide consisting of the amino acid sequence of SEQ ID NO: 1 on the expression of hair loss-related factors in hair follicle cells.
FIG. 9 shows the results of a comparative measurement of the effect of a peptide consisting of the amino acid sequence of SEQ ID NO: 1 and a peptide consisting of the amino acid sequence of SEQ ID NO: 26 on promoting the proliferation mechanism of hair follicle cells.
FIG. 10 shows the results of measuring the expression of stem cell markers after treatment with a peptide consisting of the amino acid sequence of SEQ ID NO: 1 and a peptide consisting of the amino acid sequence of SEQ ID NO: 26.
FIG. 11 shows the results of measuring the expression of differentiation markers after treatment with a peptide consisting of the amino acid sequence of SEQ ID NO: 1 and a peptide consisting of the amino acid sequence of SEQ ID NO: 26.
FIG. 12 shows the results of measuring the effect of inhibiting the expression of hair loss-related factors after treatment with a peptide consisting of the amino acid sequence of SEQ ID NO: 1 and a peptide consisting of the amino acid sequence of SEQ ID NO: 26.

### Mode for the Invention

Hereinafter, the present disclosure will be described in more detail with reference to examples. However, these examples are intended to illustrate the present disclosure by way of example and the scope of the present disclosure is not limited to these examples.

### Example 1: Synthesis of Peptides

A peptide with the amino acid sequence of SEQ ID NO: 1 listed in Table 1 below was synthesized using an automated peptide synthesizer (Milligen 9050, Millipore, USA), and the synthesized peptide was purified by C18 reversed-phase high performance liquid chromatography (HPLC) (Waters Associates, USA). The column used was an ACQUITY UPLC BEH300 C18 (2.1 mm X 100 mm, 1.7 µm, Waters Co, USA).

**[Table 1]**

| SEQ ID NO: | Sequence (N terminal → C terminal) |
|---|---|
| 1 | RQTRVERSHS |

### Example 2: Confirmation of Hair Follicle Cell Proliferation Effect

To confirm the effect of a peptide consisting of the amino acid sequence of SEQ ID NO: 1 on promoting the proliferation of hair follicle cells. Specifically, human hair follicle dermal papilla cells (HHFDPC), human hair follicle outer root sheath cells (HHFORSC), and human hair follicle germinal matrix cells (HHFGMC) were each seeded in a 96-well plate at a density of 4x10³ cells/well and then cultured for 24 hours. After replacing with serum-free mesenchymal stem cell medium, the cells were cultured for 24 hours. The above peptide was treated at concentrations of 10 µM, 50 µM, or 100 µM, respectively, and incubated at 37 °C for 72 hours. As a positive control group, 1 µM minoxidil, which is used as a hair loss therapeutic agent, 100 ng/ml of human recombinant Wnt-3a protein, and 100 ng/ml of human recombinant EGF protein were treated. After completion of culturing, the culture supernatant was removed and the cells were fixed using ethanol. After washing with PBS, staining was performed by treating with SRB solution. After washing with 1 % acetic acid, cells were observed under a microscope and destained by treating with 20 mM tris solution. Cell viability was measured by measuring absorbance at a wavelength of 560 nm using a spectrophotometer (SpectraMax iD3, Molecular Devices (USA, CA)).

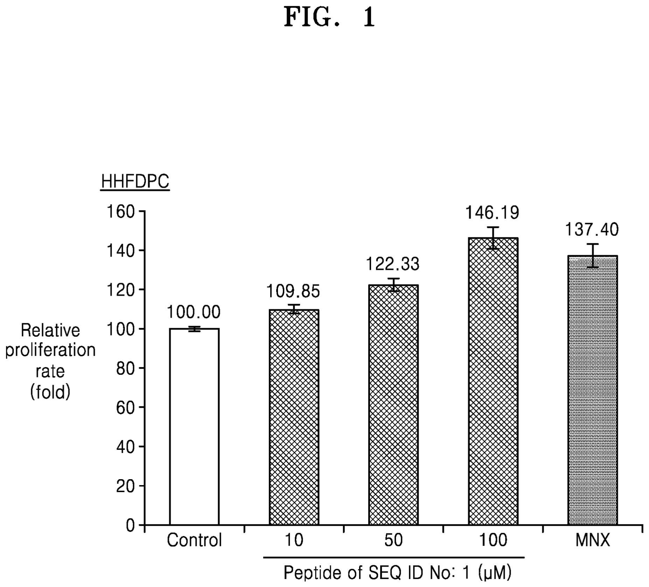

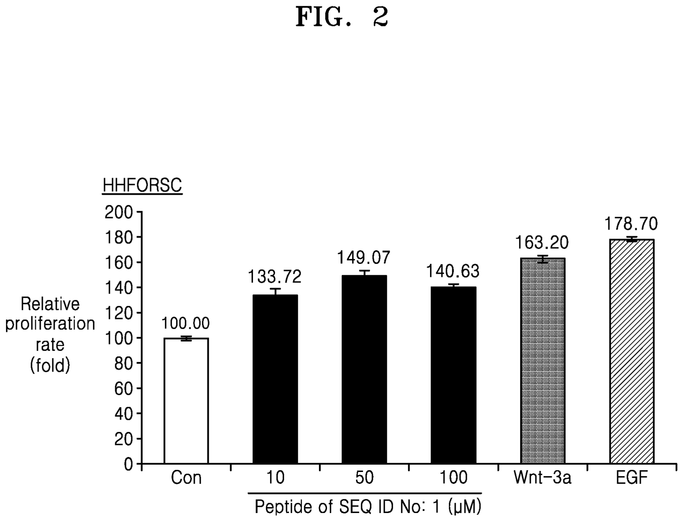

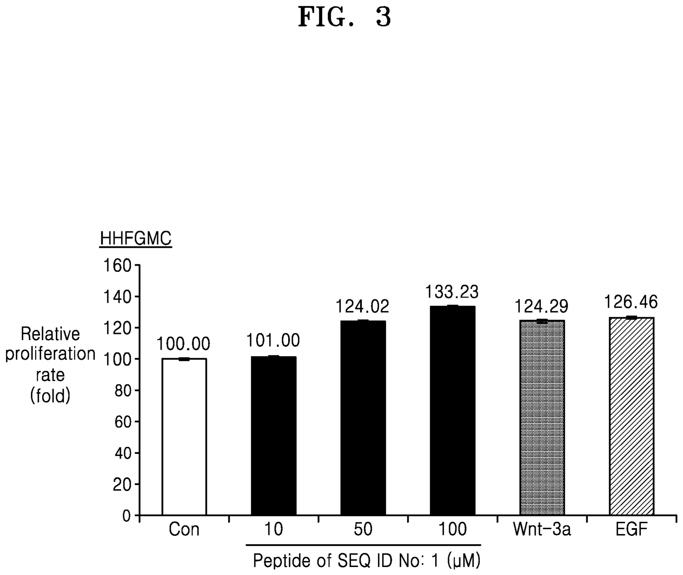

The results are illustrated in FIG. 1 to FIG. 3. FIG. 1 shows the proliferation results of human hair follicle dermal papilla cells (HHFDPC), FIG. 2 shows the proliferation results of human hair follicle outer root sheath cells (HHFORSC), and FIG. 3 shows the proliferation results of human hair follicle germinal matrix cells (HHFGMC), confirming that proliferation was induced and increased in a concentration-dependent manner in the peptide treatment group. In the case of HHFDPC, an increase of up to about 46 % was observed (FIG. 1), in the case of HHFORSC, an increase of up to about 49 % was observed (FIG. 2), and in the case of HHFGMC, an increase of up to about 33 % was observed (FIG. 3).

### Example 3: Confirmation of Effect of Promoting the Proliferation Mechanism of Hair Follicle Cells

To confirm the effect of a peptide consisting of the amino acid sequence of SEQ ID NO: 1 on promoting the proliferation mechanism of hair follicle cells. Specifically, HHFDPCs were seeded in 6-well plates at a density of 4x10⁵ cells/well and cultured for 24 hours. After replacing with serum-free mesenchymal stem cell medium, the cells were cultured for 24 hours. A peptide was treated at concentrations of 10 µM, 50 µM, or 100 µM, respectively, and incubated at 37 °C for 24 hours. As a positive control group, 10 ng/ml of recombinant human Wnt-3a protein was treated. After washing with PBS, nucleoproteins were isolated using a nucleoprotein extraction kit (Thermo scientific, USA) and quantified with BCA to prepare equal amounts of protein samples. The proteins separated by electrophoresis using a 10 % SDS-PAGE gel were transferred to a PVDF membrane. Blocking was performed at room temperature for 30 minutes using 5 % skim milk. β-catenin (cell signaling, USA) and HDAC1 (santa cruz, USA) antibodies were diluted 1:1000 in 3 % BSA and reacted with the membrane at 4 °C for 16 hours. The membrane was washed three times with 0.1 % PBS-T (0.1 % Tween-20 in PBS) for 15 minutes each. The secondary antibody was diluted 1:2000 in 5 % skim milk and reacted at room temperature for 1 hour. The membrane was then washed three times with 0.1 % PBS-T (0.1 % Tween-20 in PBS) for 15 minutes each. After treatment with ECL solution (GE Healthcare, USA), the membrane was detected.

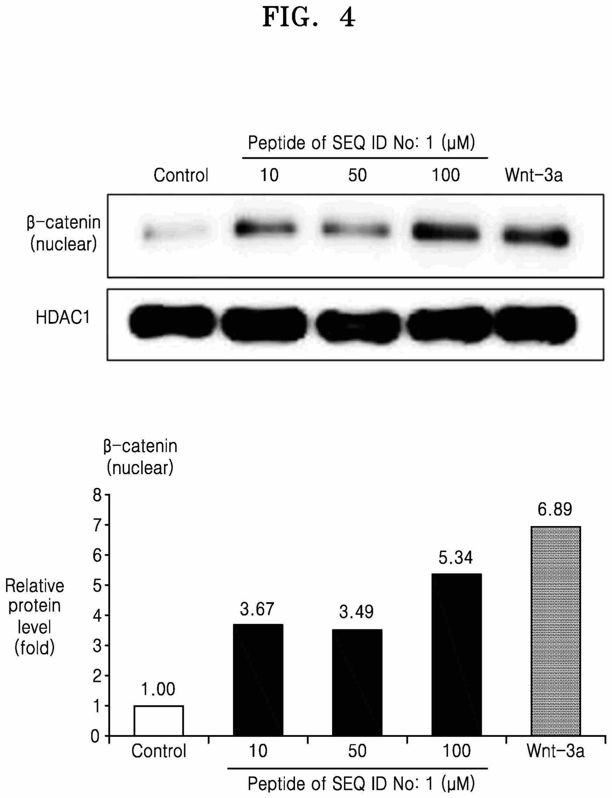

The results are illustrated in FIG. 4. From the results in FIG. 4, it may be confirmed that the expression of β-catenin increases when treated with peptide, and that nuclear translocation of β-catenin from the cytoplasm to the nucleus increases by up to 5.34 times by peptide treatment.

### Example 4: Confirmation of Effect of Promoting Hair Follicle Cell Activity

To confirm the effect of a peptide consisting of the amino acid sequence of SEQ ID NO: 1 on promoting hair follicle cell activity. HHFDPCs were seeded in 6-well plates at a density of 4x10⁵ cells/well and cultured for 24 hours. After replacing with serum-free mesenchymal stem cell medium, the cells were cultured for 24 hours. A peptide was treated at concentrations of 10 µM, 50 µM, or 100 µM, respectively, and incubated at 37 °C for 24 hours. As a positive control group, 10 ng/ml of recombinant human Wnt-3a protein was treated. After washing with PBS, RNA was isolated by treating with 300 µL of easy blue (intron, Korea). After RNA quantification, cDNA synthesis was performed using a cDNA synthesis kit (enzynomics, Korea). PCR was performed using PCR pre-mix (enzynomics, Korea) using the Lef-1, Cyclin D1, and c-Myc primers shown in Table 2. Electrophoresis was performed on a 1.5 % agarose gel and band detection was performed using a Bio-Rad gel image system.

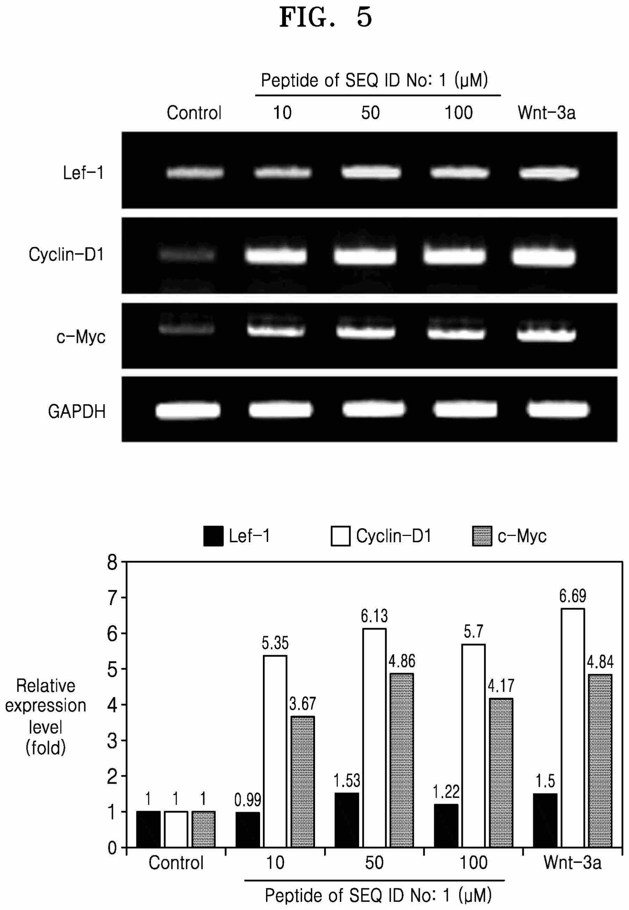

The results are illustrated in FIG. 5. From the results in FIG. 5, it was confirmed that the expression of Lef-1, c-Myc, and Cyclin D1, which are factors related to the activity and growth of HHFDPC cells, was increased by peptide treatment. In the case of Lef-1, it was confirmed that the expression increased by up to 1.53 times compared to the control group, in the case of Cyclin D1 by up to 6.13 times, and in the case of c-Myc by up to 4.86 times.

**[Table 2]**

| Primer | Sequence | SEQ ID NO: |
|---|---|---|
| LEF-1 forward | (5') CCAGCTATTGTAACACCTCA (3') | 2 |
| LEF-1 reverse | (5') TTCAGATGTAGGCAGCTGTC (3') | 3 |
| c-Myc forward | (5') ACCAGCAGCGACTCTGAGGAGGAAC (3') | 4 |
| c-Myc reverse | (5') TGACCCTCTTGGCAGCAGGATAGTCC (3') | 5 |
| Cyclin D1 forward | (5') ACCTTCGTTGCCCTCTGTGCCACAGAT G (3') | 6 |
| Cyclin D1 reverse | (5') AGGCCCGGAGGGCAGTCCGGGT (3') | 7 |
| GAPDH forward | (5') GGAGCCAAAAGGGTCATCAT (3') | 8 |
| GAPDH reverse | (5') GTGATGGCATGGACTGTGGT (3') | 9 |

### Example 5: Confirmation of Effect of Inducing Differentiation of Stem Cells

To confirm the differentiation-inducing effect of a peptide consisting of the amino acid sequence of SEQ ID NO: 1 on stem cells. HHFSCs were seeded in 6-well plates at a density of 1x10⁵ cells/well and cultured for 24 hours. After replacing with serum-free mesenchymal stem cell medium, the cells were cultured for 24 hours. A peptide was treated at concentrations of 10 µM, 50 µM, or 100 µM, respectively, and incubated at 37 °C for 72 hours. As a positive control group, recombinant human Wnt-3a protein was treated at 10 ng/ml and 100 ng/ml, respectively. After washing with PBS, RNA was isolated by treating with 300 µL of easy blue (intron, Korea). After RNA quantification, cDNA synthesis was performed using a cDNA synthesis kit (enzynomics, Korea). PCR was performed using PCR pre-mix (enzynomics, Korea) using the primers shown in Table 3. Electrophoresis was performed on a 1.5 % agarose gel and band detection was performed using a Bio-Rad gel image system.

**[Table 3]**

| Primer | Sequence | SEQ ID NO: |
|---|---|---|
| CD34 forward | (5') CTGCATGCAAGAGTGACACC (3') | 10 |
| CD34 reverse | (5') GACATTGCCCCTTCCCTTTG (3') | 11 |
| Lhx-2 forward | (5') AAGCTGCAACGAAAACGACG (3') | 12 |
| Lhx-2 reverse | (5') GGTGGGGCTAGTCAAGTTCTG (3') | 13 |
| Keratin 15-Forward | (5') CCAGCAAGACGGAGATCACA (3') | 14 |
| Keratin 15-Reverse | (5') GAAGAGGCTTCCCTGATGGC (3') | 15 |
| Alpha-SMA forward | (5') CCTATCCCCGGGACTAAGAC (3') | 16 |
| Alpha-SMA reverse | (5') ATGCTCTTCAGGGGCAACAC (3') | 17 |
| Corin forward | (5') CAGTGGAATCTGCATCCCCG (3') | 18 |
| Corin reverse | (5') CAGCGATGCTCTGTTGTGG (3') | 19 |
| Versican forward | (5') CCGTTCTCCCCAGGAAAACTT (3') | 20 |
| Versican reverse | (5') TGCAGCGATCAGGTCGTTTA (3') | 21 |
| Vimentin forward | (5') TGGACCAGCTAACCAACGAC (3') | 22 |
| Vimentin reverse | (5') GTCATTGTTCCGGTTGGCAG (3') | 23 |
| Nexin-1 forward | (5') CCCAGCCAACCTATGAGGAG (3') | 24 |
| Nexin-1 reverse | (5') CTTGAGGAGACCAGCACCAC (3') | 25 |

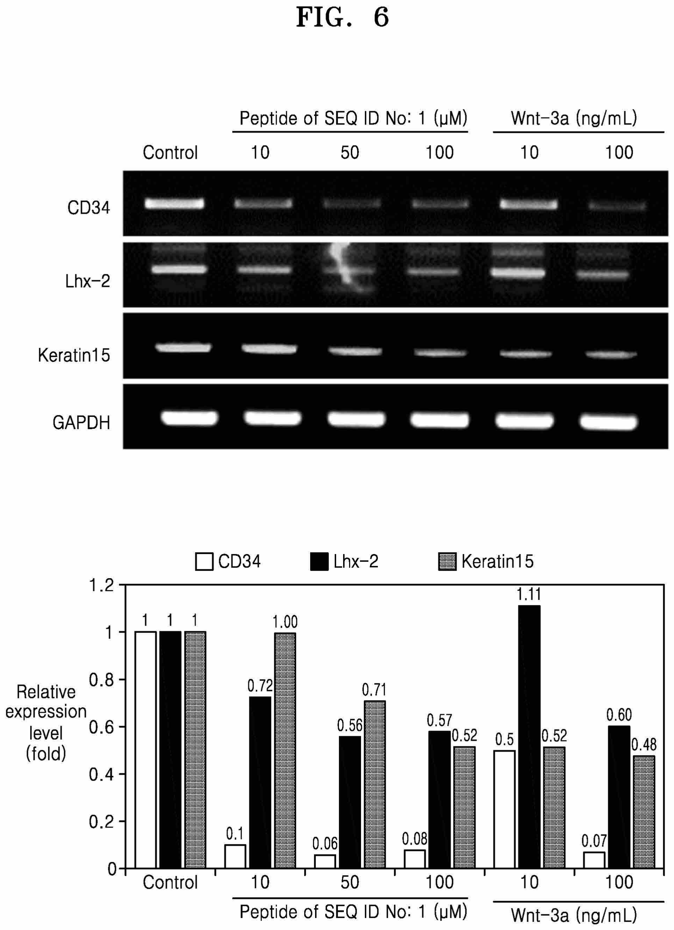

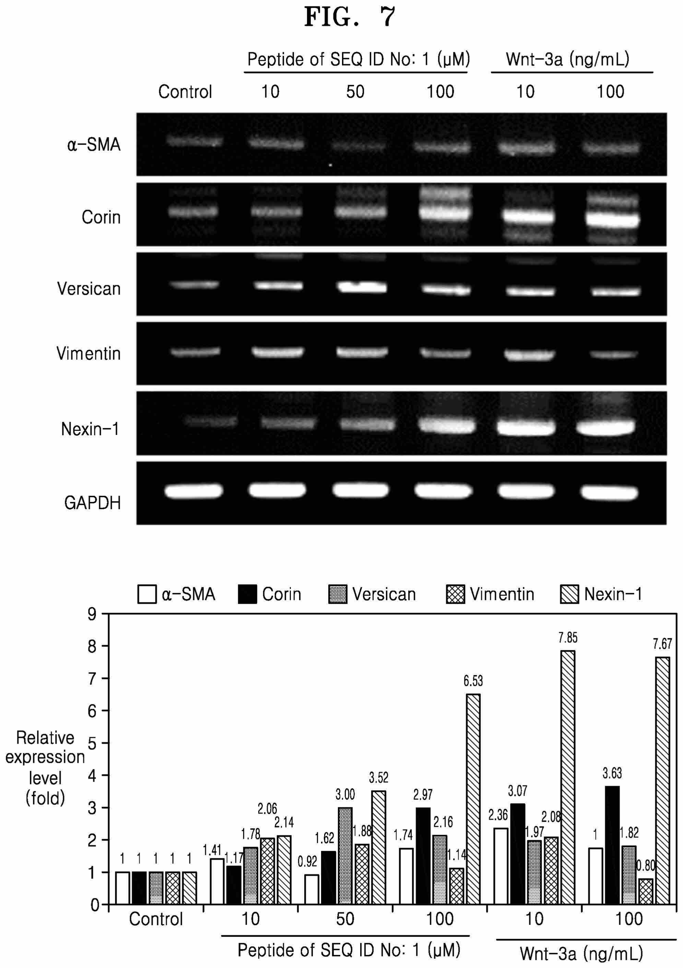

FIG. 6 shows the results of measuring stem cell markers, and FIG. 7 shows the results of measuring differentiation markers. From the results in FIG. 6, it may be confirmed that the expression of stem cell markers CD34, Lhx-2, and Keratin15 is clearly reduced. On the other hand, from the results of FIG. 7, it may be confirmed that the expression of all differentiation markers, alpha-SMA, Corin, Versican, Vimentin, and Nexin-1, is significantly increased. From this, it was confirmed that the peptide having the amino acid sequence of SEQ ID NO: 1 induces the proliferation of stem cells and induces differentiation so that they may go to hair roots, thereby enabling the creation of new hair follicles.

### Example 6: Confirmation of Effect of Inhibiting Expression of Hair Loss-related Factors in Hair Follicle Cells

To confirm whether a peptide consisting of the amino acid sequence of SEQ ID NO: 1 inhibits the expression of DKK-1, a hair loss-related factor, in hair follicle cells. HHFDPCs were seeded in 6-well plates at a density of 4x10⁵ cells/well and cultured for 24 hours. After replacing with serum-free mesenchymal stem cell medium, the cells were cultured for 24 hours. The peptides were treated at concentrations of 10 µM, 50 µM, or 100 µM, respectively, and simultaneously treated with DHT, an inducer of hair loss protein DKK-1, at a concentration of 100 nM. As a positive control group, 5 µM of finasteride, a drug that inhibits androgens, was treated. DHT is known as a hair loss hormone that increases the expression of DKK-1 (Dickkopf-related protein 1), a hair loss-inducing factor, by activating androgen receptors. After culturing at 37 °C for 24 hours, the cells were washed with PBS, treated with lysis buffer to prepare cell lysates, and equal amounts of protein samples were prepared by BCA quantification. Electrophoresis was performed using a 10 % SDS-PAGE gel. Proteins separated by SDS PAGE were transferred to a PVDF membrane. Blocking was performed at room temperature for 30 minutes using 5 % skim milk. DKK-1 (cell signaling, USA) antibody was diluted 1:1000 in 3 % BSA and reacted with the membrane at 4 °C for 16 hours. The membrane was then washed three times with 0.1 % PBS-T (0.1 % Tween-20 in PBS) for 15 minutes each. The secondary antibody was diluted 1:2000 in 5 % skim milk and reacted at room temperature for 1 hour. The membrane was then washed three times with 0.1 % PBS-T (0.1 % Tween-20 in PBS) for 15 minutes each. After treatment with ECL solution (GE Healthcare, USA), protein band detection and densitometry were performed using ImageQuant 800 (Amersham, USA).

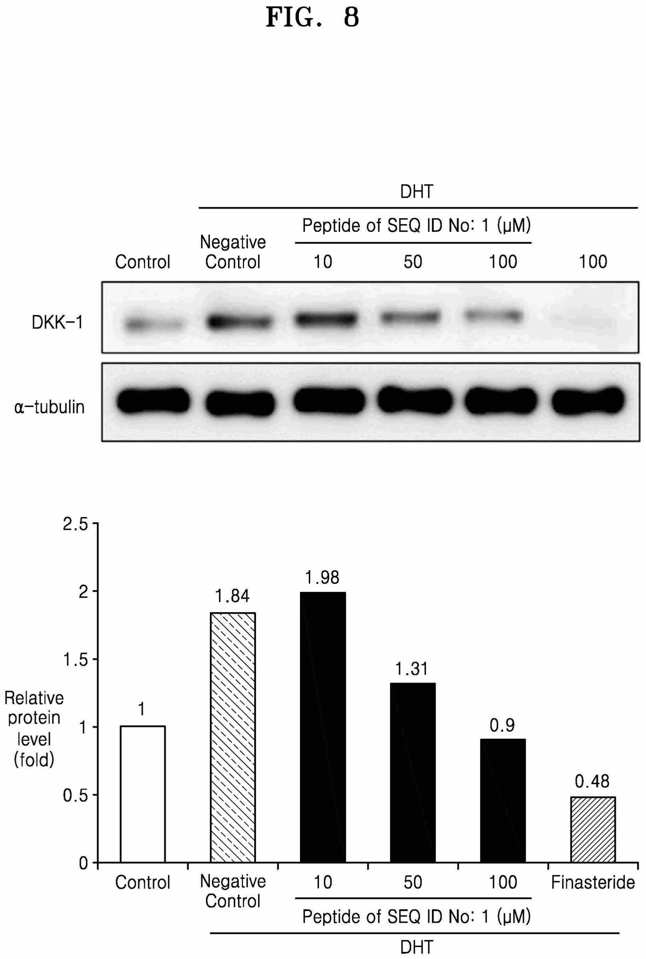

The results are illustrated in FIG. 8. From the results of FIG. 8, it was possible to confirm the effect of inhibiting DKK-1 expression induced by DHT after peptide treatment. In the high-concentration peptide treatment group, it was confirmed that the DKK-1 expression level was reduced by about 50 % compared to the negative control group (DHT treatment group).

### Example 7: Comparison of Effects of Promoting Proliferation Mechanism of Hair Follicle Cells

The proliferation-promoting effects of a novel peptide consisting of the amino acid sequence of SEQ ID NO: 1 and a peptide consisting of the amino acid sequence of SEQ ID NO: 26 disclosed in KR10-1209117 (Table 4) on hair follicle cell proliferation were compared. The experiment was conducted in the same manner as described in Example 3, and the cell proliferation effect was measured after treating HHFDPC with peptides at different concentrations.

**[Table 4]**

| SEQ ID NO: | Sequence (5'→3') |
|---|---|
| 26 | RQTRVQRSHS |

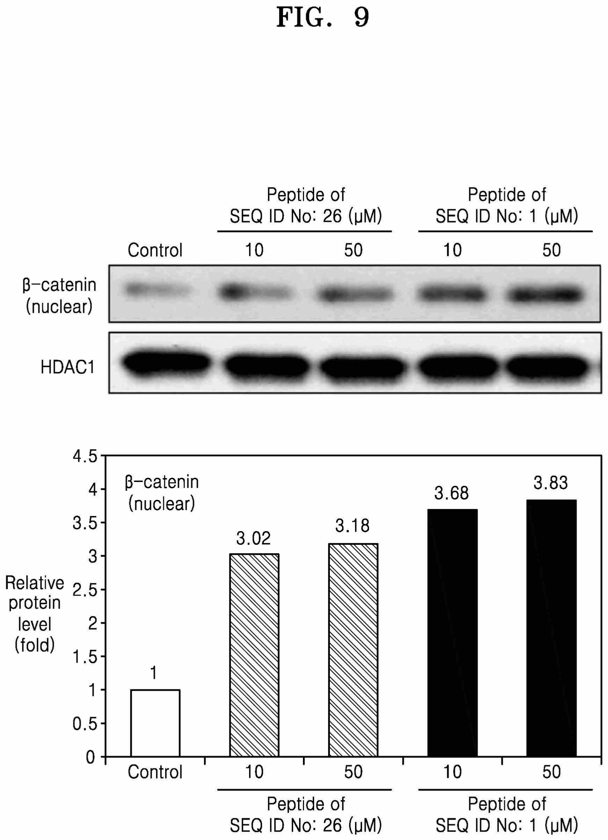

The results are illustrated in FIG. 9. From the results in FIG. 9, it may be confirmed that both a peptide consisting of the amino acid sequence of SEQ ID NO: 26 and a novel peptide consisting of the amino acid sequence of SEQ ID NO: 1 promote the translocation of β-catenin from the cytoplasm to the nucleus in a concentration-dependent manner. In addition, when comparing the activities of the two peptides, it may be confirmed that the novel peptide exhibits significantly higher proliferation mechanism promoting activity than the known peptide when treated at the same concentration.

### Example 8: Comparison of Effects of Inducing Differentiation of Stem Cells

The differentiation-inducing effects of a novel peptide consisting of the amino acid sequence of SEQ ID NO: 1 and a peptide consisting of the amino acid sequence of SEQ ID NO: 26 on stem cells were compared. The experiment was conducted in the same manner as described in Example 5, and the differentiation-inducing effect was confirmed by treating HHFSCs with peptides at different concentrations.

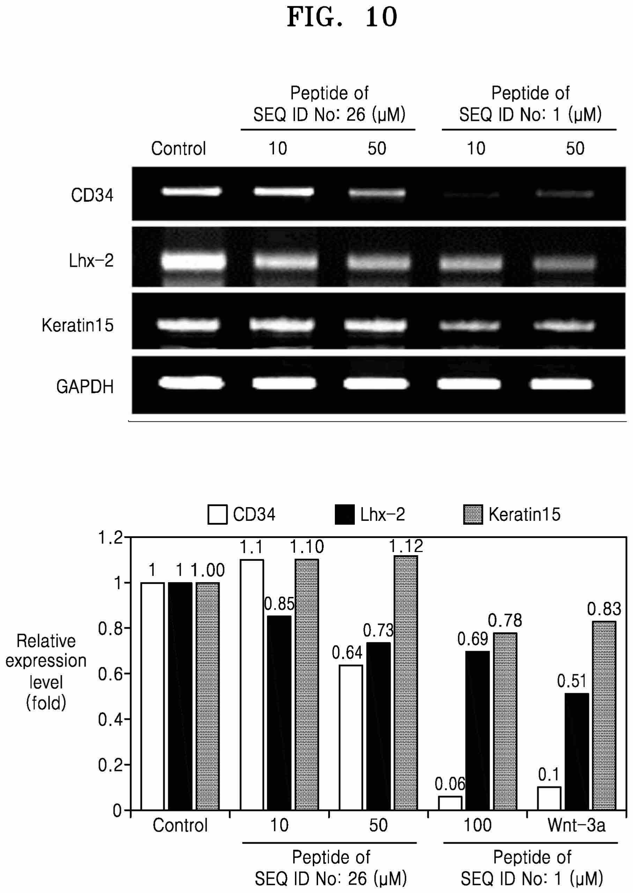

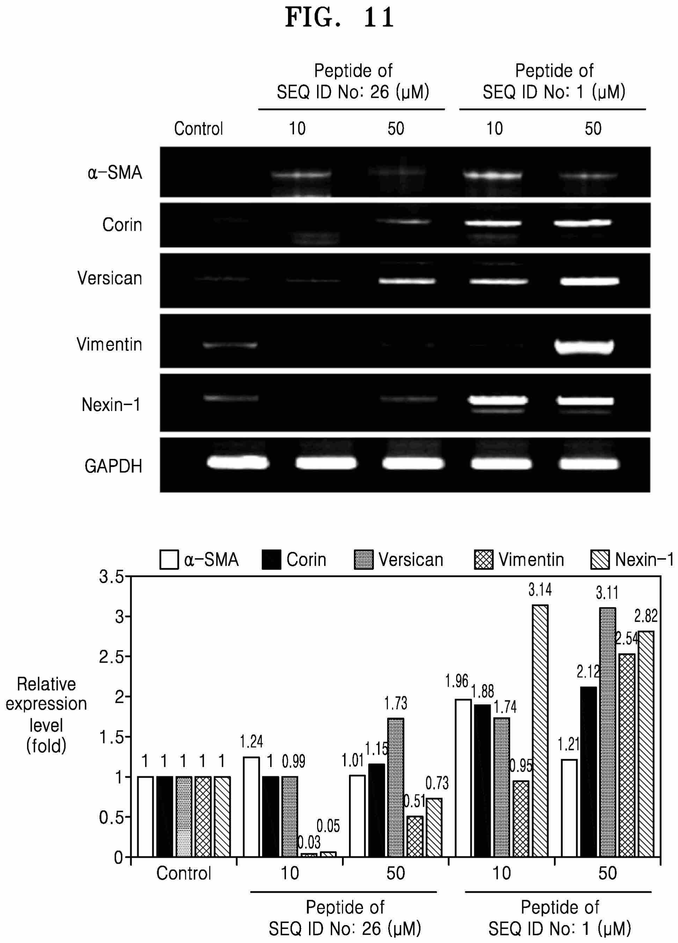

The results are illustrated in FIG. 10 and FIG. 11. FIG. 10 shows the results of measuring stem cell markers, and FIG. 11 shows the results of measuring differentiation markers. From the results in FIG. 10, it may be confirmed that the expression of stem cell markers CD34, Lhx-2, and Keratin15 is significantly reduced when treated with the two peptides. And when comparing the activities of the two peptides, it may be confirmed that the expression of stem cell markers is reduced more significantly by the novel peptide than by the known peptide when treated at the same concentration. In addition, from the results of FIG. 11, it may be confirmed that the expression of all differentiation markers, alpha-SMA, Corin, Versican, Vimentin, and Nexin-1, significantly increases when treated with the two peptides. And when comparing the activities of the two peptides, it may be confirmed that the expression of differentiation markers is significantly increased in the novel peptide compared to the known peptide when treated with the same concentration. Therefore, it may be confirmed that the novel peptide induces the proliferation of stem cells and induces their differentiation to the hair follicles more effectively than the known peptide, thereby enabling the creation of new hair follicles.

### Example 9: Comparison of Effect of Inhibiting Expression of Hair Loss-related Factors in Hair Follicle Cells

The effect of inhibiting the expression of hair loss-related factors was compared between the novel peptide consisting of the amino acid sequence of SEQ ID NO: 1 and the peptide consisting of the amino acid sequence of SEQ ID NO: 26. The experiment was conducted in the same manner as described in Example 6, and it was confirmed whether the expression of DKK-1, a hair loss-related factor, was inhibited after treating HHFDPC with peptides at different concentrations.

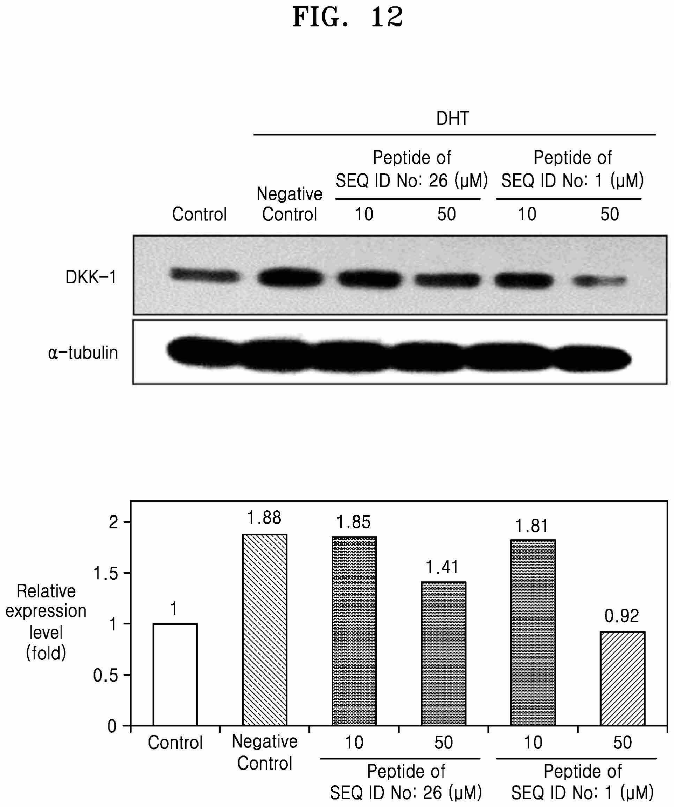

The results are illustrated in FIG. 12. From the results of FIG. 12, it may be confirmed that both a peptide consisting of the amino acid sequence of SEQ ID NO: 26 and a novel peptide consisting of the amino acid sequence of SEQ ID NO: 1 exhibit an inhibitory effect on DKK-1 expression in a concentration-dependent manner. In addition, when comparing the inhibitory effects of the two peptides, it may be confirmed that the novel peptide has a significantly higher ability to inhibit the expression of DKK-1 than the known peptide when treated with the same concentration.

### Formulation Example 1. Preparation of Peptide Nanosomes

50 mg of the peptide of Example 1 above was dissolved in 500 ml of distilled water by sufficient stirring. The composite solution was mixed with 5 g of lecithin, 0.3 ml of sodium oleate, 50 ml of ethanol, and a small amount of oil, and the total amount was adjusted to 1 L with distilled water, and the mixture was then emulsified using a high pressure microfluidizer to produce peptide nanosomes with a size of about 100 nm.

### Formulation Example 2. Lotion

A lotion including the peptide according to an example and consisting of the following composition was prepared using a method known in the art.

**[Table 5]**

| Component | Content (wt%) |
|---|---|
| Peptide | 1.0 |
| 1,3-Butylene glycol | 6.0 |
| Glycerin | 4.0 |
| PEG 1500 | 1.0 |
| Sodium hyaluronate | 1.0 |
| Polysorbate 20 | 0.5 |
| Ethanol | 8.0 |
| Benzophenone-9 | 0.05 |
| Preservatives, colorants | As required |
| Fragrance | As required |
| Purified water | Remainder |
| Total | 100 |

### Formulation Example 3. Nutrition Cream

A nutrition cream including the peptide according to an example and consisting of the following composition was prepared using a method known in the art.

**[Table 6]**

| Component | Content (wt%) |
|---|---|
| Peptide | 2.0 |
| Glycerin | 10.0 |
| 1,3-Butylene glycol | 5.0 |
| PEG 1500 | 2.0 |
| Allantoin | 0.1 |
| DL-Panthenol | 0.3 |
| EDTA-2Na | 0.02 |
| Hydroxyethyl cellulose | 0.1 |
| Sodium hyaluronate | 8.0 |
| Carboxyvinyl polymer | 0.2 |
| Triethanolamine | 0.18 |
| Octyldodeceth-16 | 0.4 |
| Ethanol | 6 |
| Preservatives, colorants | As required |
| Fragrance | As required |
| Purified water | Remainder |
| Total | 100 |

The foregoing description of the present disclosure is for illustrative purposes only, and one that has ordinary skill in the art to which the present disclosure belongs will understand that it may be readily adapted to other specific forms without altering the technical ideas or essential features of the present disclosure. Therefore, the examples described above should be understood in all respects as illustrative and not restrictive.

## Claims

1. A peptide consisting of the amino acid sequence of SEQ ID NO: 1.

2. The peptide of claim 1, wherein an N-terminus of the peptide is bound to any one protecting group selected from the group consisting of an acetyl group, a fluoreonylmethoxycarbonyl group, a formyl group, a palmitoyl group, a myristyl group, a stearyl group, a butoxycarbonyl group, an allyloxycarbonyl group, and polyethylene glycol (PEG).

3. The peptide of claim 1, wherein a C-terminus of the peptide is bound to any one protecting group selected from the group consisting of an amino group (-NH₂), a tertiary alkyl group, and azide (-NHNH₂).

4. The peptide of claim 1, wherein the peptide exhibits any one or more characteristics selected from the following:
(a) promotion of proliferation of human hair follicle dermal papilla cells;
(b) promotion of proliferation of human hair follicle outer root sheath cells;
(c) promotion of proliferation of human hair follicle germinal matrix cells;
(d) activation of β-catenin;
(e) increased expression of lymphoid enhancer-binding factor-1 (LEF-1), Cyclin D1, or c-Myc;
(f) promotion of differentiation of human hair follicle stem cells; and
(g) inhibition of expression of Dickkopf-related protein 1 (DKK-1).

5. A cosmetic composition for promoting hair growth or improving hair loss, comprising the peptide of any one of claims 1 to 4 as an active ingredient.

6. A pharmaceutical composition for preventing or treating hair loss, comprising the peptide of any one of claims 1 to 4 as an active ingredient.
